# EUROPÄISCHE PATENTSCHRIFT

(11) **EP 2 819 534 B1**
(45) Veröffentlichungstag und Bekanntmachung des Hinweises auf die Patenterteilung: **14.09.2016**
(21) Anmeldenummer: 13714194.1
(22) Anmeldetag: 01.03.2013
(51) Int. Cl.: A61M 11/04, A24F 47/00, A61M 15/06

(54) **PASSIVE INHALATIONSEINRICHTUNG**
PASSIVE INHALATION DEVICE
DISPOSITIF D'INHALATION PASSIF

(30) Priorität: 01.03.2012 DE 102012004179
(43) Veröffentlichungstag der Anmeldung: 07.01.2015
(73) Patentinhaber: Similtrade GmbH, 6343 Rotkreuz (CH)
(72) Erfinder: SPÖRRI, Anton, 6353 Weggis (CH); SPÖRRI, Markus, 6314 Unterägeri (CH); BÜTLER, Beat, 6006 Luzern (CH)
(74) Vertreter: Weigel, Matthias
(86) Internationale Anmeldenummer: PCT/EP2013/000607
(87) Internationale Veröffentlichungsnummer: WO 2013/127538

(56) Entgegenhaltungen:
- EP-A1- 0 364 805
- EP-A1- 1 609 376
- EP-A2- 0 198 268
- WO-A1-88/01884
- DE-A1-102004 027 961
- DE-A1-102010 000 043
- DE-U1-202007 004 678
- GB-A- 873 410
- US-A- 3 320 953
- US-A- 3 521 643
- US-A- 4 284 089
- US-A- 5 501 236
- US-A- 5 799 663
- US-A1- 2011 036 363

## Beschreibung

Die Erfindung betrifft eine passive Inhalationseinrichtung nach dem Oberbegriff des Anspruchs 1, insbesondere eine Inhalationseinrichtung für pharmazeutische Wirkstoffe, eine rauchfreie kalte Zigarette, Zigarre oder Pfeife, zur Abgabe mindestens einer bei Raumtemperatur zumindest teilweise in die Gasphase übergehenden flüchtigen Substanz, bei der die für die Freisetzung der flüchtigen Substanz erforderliche Erwärmung im wesentlichen passiv durch die Körpertemperatur des Benutzers erfolgt.

Bereits seit langem ist es bekannt, dass Benutzer flüchtige Substanzen mit Hilfe sogenannter Inhalationseinrichtungen inhalieren. Die Inhalationseinrichtung weist hierzu ein Depot auf, aus dem die Substanz freigesetzt und an einem Luftstrom abgegeben wird, der vom Benutzer durch die Inhalationseinrichtung gesogen wird. Seit geraumer Zeit werden derartige Inhalationseinrichtungen insbesondere auch für das Inhalieren von pharmazeutisch wirksamen Substanzen zu therapeutischen oder diagnostischen Zwecken verwendet, beispielsweise zur Behandlung von Hals- und Rachenschmerzen oder auch zur Behandlung von Lungenerkrankungen. Der Vorteil hierbei ist, dass mit vergleichsweise geringen technischen Mitteln, eine definierte Abgabe der als Wirkstoffe dienenden flüchtigen Substanzen möglich ist. Um eventuell unangenehme Begleiterscheinungen zu eliminieren, ist es auch möglich, neben den Wirkstoffen als zusätzliche Substanzen Aromastoffe oder Geschmacksstoffe beimengen.

Anstelle pharmazeutisch wirksamer Substanzen können als Wirkstoffe auch Nikotin oder nikotinhaltige Verbindungen verwendet werden, die vom Benutzer konsumiert werden. Der maßgebliche Vorteil hierin liegt in der Tatsache, dass der Konsum erfolgen kann, ohne dass hierbei Tabak unter Rauchentwicklung verbrannt werden muss. Diese sogenannten rauchfreien Zigaretten, Zigarren oder Pfeifen sind als Inhalationseinrichtungen ausgebildet, mit deren Hilfe Wirkstoffe, wie Nikotin oder nikotinhaltige Verbindungen, und auch Aromastoffe gezielt und definiert freigesetzt werden, die anschließend vom Benutzer inhaliert werden. Unter dem Begriff "nikotinhaltige Verbindung" werden in diesem Zusammenhang insbesondere Nikotinsalze, Nikotinderivate sowie nikotinhaltige Tabakinhaltsstoffe verstanden.

Bei den sogenannten rauchfreien Zigaretten, Zigarren oder Pfeifen, wird zwischen zwei wesentliche Grundprinzipien unterschieden, der sogenannten "passiven Zigarette" oder "kalten Zigarette", bei der der Wirkstoff lediglich durch die Raumtemperatur oder die Körpertemperatur des Benutzers aus dem Depot freigesetzt wird, oder der sogenannten "aktiven Zigarette" oder "elektronischen Zigarette", bei der mit Hilfe elektrisch betriebener Heizeinrichtungen oder mit Hilfe elektrisch betriebener Zerstäuber der Wirkstoff aus dem Depot abgegeben wird.

Die vorliegende Erfindung betrifft das erste Grundprinzip, bei dem die bei Raumtemperatur zumindest teilweise flüchtige Substanz mit Hilfe der Körpertemperatur des Benutzers aus dem Depot freigesetzt wird, die Einrichtung also passiv betrieben wird

So ist eine als passive Inhalationseinrichtung dienende "kalte Zigarette" bekannt, bei der das in einem Strömungskanal der "kalten Zigarette" angeordnete Wirkstoff-Depot mit einem Wärmeübertragungselement in Berührung steht. Bei Verwendung hält der Benutzer die "kalte Zigarette" im Bereich des nach außen hin zugänglichen Wärmeübertragungselementes und erwärmt mit seiner über die Finger übertragenen Körpertemperatur den im Depot gespeicherten Wirkstoff, im vorliegenden Fall Nikotin bzw. nikotinhaltige Verbindungen. Die Wärmeleitfähigkeit sowie die Wärmekapazität des Wärmeübertragungselementes sind dabei so ausgelegt, dass die Körpertemperatur des Benutzers ausreicht, das Depot soweit zu erwärmen, dass der im Depot gespeicherte Wirkstoff freigesetzt wird. Da das Depot im Strömungskanal der "kalten Zigarette" angeordnet ist, durch den der Benutzer Luft ansaugt, wird der aus dem Depot freigesetzte Wirkstoff an den Luftstrom abgegeben.

Problematisch bei den derzeit bekannten passiven Inhalationseinrichtungen ist, dass diese nur in kleinen Stückzahlen hergestellt werden, die bekannten passiven Inhalationseinrichtungen für eine Produktion in große Stückzahlen jedoch nicht ausgelegt sind.

Ausgehend von diesem Stand der Technik ist es Aufgabe der Erfindung eine passive Inhalationseinrichtung bereitzustellen, die einfach und in großen Stückzahlen herzustellen ist.

Erfindungsgemäß wird diese Aufgabe durch eine passive Inhalationseinrichtung mit den Merkmalen des Anspruchs 1 und insbesondere dadurch gelöst, dass der Grundkörper zwei den Strömungskanal abschnittsweise bildende Hülsen aufweist, zwischen deren einander zugewandten Stirnseiten das Depot angeordnet ist, dass das als Teil des Grundkörpers ausgebildete Wärmeübertragungselement eine einen weiteren Abschnitt des Strömungskanals begrenzende Wärmeübertragungshülse ist, in der das Depot gehalten ist, und dass die Wärmeübertragungshülse die beiden Hülsen starr miteinander verbindet und an diesen gasdicht befestigt ist.

Ein wesentlicher Vorteil der erfindungsgemäßen Inhalationseinrichtung besteht in dem modularen Aufbau aus einzelnen, für sich betrachtet vergleichsweise einfach gestalteten Komponenten.

So ist zunächst die Verwendung der Wärmeübertragungshülse bei der Erfindung hervorzuheben. Einerseits dient die Wärmeübertragungshülse als Aufnahme für das Depot, andererseits dient die Wärmeübertragungshülse gleichzeitig als Verbindungsglied zwischen den beiden Hülsen.

Ihre Funktion als Aufnahme für das Depot erleichtert wesentlich die Herstellung der erfindungsgemäßen Inhalationseinrichtung. So kann das Depot entweder vorab mit der flüchtigen Substanz befüllt und in befülltem Zustand in die Wärmeübertragungshülse eingesetzt werden. Oder das Depot wird in noch unbefülltem Zustand in die Wärmeübertragungshülse eingesetzt und erst nach dem Einsetzen in die Wärmeübertragungshülse mit der Substanz befüllt. Dies ist insbesondere dann von Vorteil, wenn das Depot mit einer Flüssigkeit als Substanz befüllt wird. In diesem Fall dient die Wärmeübertragungshülse gleichzeitig als Befüllungsreservoir, in dem die aufrecht stehende Wärmeübertragungshülse oberhalb des Depots mit der Substanz befüllt wird und das Depot dann beispielsweise über Kapillarkräfte die in die Wärmeübertragungshülse eingefüllte Flüssigkeit aufnimmt.

Ferner ist es auf sehr einfache Weise möglich, je nach Dosierung oder Art der Substanz unterschiedliche Depotarten in die Wärmeübertragungshülse einzusetzen. Dies hat keinerlei Einfluss auf das Endprodukt. Des weiteren können auch verschiedene Wärmeübertragungshülsen aus Werkstoffen mit sich voneinander unterscheidenden Wärmeleitfähigkeiten und Wärmekapazitäten verwendet werden, um unterschiedliche Freisetzungsraten oder Dosierungen für verschiedene Substanzen zu realisieren. Hierdurch wird erreicht, dass die üblicherweise vollautomatisierten Produktionsanlagen auf unterschiedliche Produkte nicht angepasst werden müssen.

In gleicher Weise wird durch den modularen Aufbau, insbesondere die Verwendung der beiden mit der Wärmeübertragungshülse fest verbundenen Hülsen erreicht, dass die gesamte Inhalationseinrichtung an den jeweiligen Anwendungszweck einfach angepasst werden kann. Die beiden Hülsen bieten definierte Befestigungsflächen oder Befestigungsabschnitte, an denen die Wärmeübertragungshülse sicher und gasdicht befestigt werden kann. Ferner erlauben die beiden Hülsen ein einfaches Anpassen der Inhalationseinrichtung hinsichtlich ihres Erscheinungsbildes. Soll die Inhalationseinrichtung beispielsweise das Erscheinungsbild einer herkömmlichen Zigarette haben, können auf die beiden Hülsen entsprechende Papierhülsen aufgeschoben und mit diesen fest verbunden werden. Gleiches gilt auch für die Gestaltung der Inhalationseinrichtung als "Zigarre" oder "Pfeife".

Die verschiedenen Komponenten der erfindungsgemäßen Inhalationseinrichtung, nämlich die beiden Hülsen, die Wärmeübertragungshülse sowie das Depot, lassen sich aufgrund deren einfachen Gestaltung in großen Stückzahlen leicht und unabhängig voneinander aus unterschiedlichsten Materialen fertigen. Hierdurch ergibt sich eine große Variantenvielfalt, die sich bei den bisher bekannten passiven Inhalationseinrichtungen aufgrund deren komplexen Aufbaus nicht ergibt.

Die Wärmeübertragungshülse hat eine hohlzylindrische Form mit gleichbleibendem Querschnitt über ihre gesamte Länge, kann jedoch gegebenenfalls im Querschnitt eine oval oder auch mehreckig Querschnittsform aufweisen. Für eine optimale Wärmeübertragung ist es ferner von Vorteil, wenn die Innenoberfläche der Wärmeübertragungshülse mit der gesamten Umfangsoberfläche des Depots in Berührung steht, um eine maximale Wärmeübertragung von der Wärmeübertragungshülse auf das Depot sicherzustellen.

Weitere Vorteile der Erfindung ergeben sich aus der nachfolgenden Beschreibung, den Unteransprüchen und den Zeichnungen.

Bevorzugt ist die Wärmeübertragungshülse zumindest abschnittsweise aus einem Material mit sehr hoher Wärmeleitfähigkeit gefertigt, beispielsweise Aluminium oder einer Aluminiumlegierung. Insbesondere der Einsatz von Aluminium oder einer Aluminiumlegierung als Werkstoff für die Wärmeübertragungshülse ist von besonderem Vorteil, da diese Werkstoffe einerseits verglichen mit anderen Werkstoffen einfach zu verarbeiten sind, während sie andererseits vergleichsweise kostengünstig sind.

Um eine besonders gute mechanische Verbindung zwischen jeder der beiden Hülsen und der Wärmeübertragungshülse zu ermöglichen, weisen die beiden Hülsen bei einer besonders bevorzugten Ausführungsform jeweils einen Bund größeren Durchmessers auf, welcher in einen das freie Ende der jeweiligen Hülse bildenden Absatz kleineren Durchmessers übergeht. Zum Befestigen an der Wärmeübertragungshülse, wird jede Hülse mit ihrem Absatz in die Wärmeübertragungshülse soweit eingeführt, bis sich die Hülse mit ihrem Bund an der jeweiligen Stirnseite der Wärmeübertragungshülse vorzugsweise abstützt. Der Absatz kleineren Durchmessers bildet somit einen definierten Befestigungsabschnitt, mit dem die Hülse sicher in der Wärmeübertragungshülse gehalten ist und mit dieser durch geeignete Befestigungsmethoden, wie Einpressen, Verkleben, oder auch Verschweißen, fest verbunden werden kann. Ferner ist es auch möglich, den Absatz an seiner Stirnseite mit einer umlaufenden Fase zu versehen, die das Einführen des Absatzes in die Wärmeübertragungshülse insbesondere in automatisierten Abläufen zusätzlich erleichtert. Die Außenabmessung des Absatzes und die Innenabmessung der Wärmeübertragungshülse sind dabei so gewählt, dass zwischen beiden Komponenten eine Spielpassung oder auch eine Übergangspassung ausgebildet ist. Auf diese Weise wird bereits beim Zusammensetzen ein sicherer und definierter Halt zwischen den beiden Komponenten gewährleistet. In gleicher Weise unterstützt auch der als Anschlag dienende Bund eine exakte Positionierung zwischen der Wärmeübertragungshülse und der Hülse. Der Bund begrenzt nicht nur die Einführbewegung der Hülse in die Wärmeübertragungshülse, sondern führt darüber hinaus nach dem Zusammensetzen zu einer definierten Relativlage zwischen der Wärmeübertragungshülse und der Hülse selbst, wodurch insbesondere nachfolgende automatisiert ablaufende Fertigungsschritte weiter erleichtert sind.

Damit die erfindungsgemäße Inhalationseinrichtung nach außen hin besonders gut gasdicht abgedichtet ist, wird bei der zuvor beschrieben Ausführungsform ferner vorgeschlagen, an jedem Absatz zumindest eine umlaufende Dichtungskontur vorzusehen, mit welcher der Absatz an der Innenoberfläche der Wärmeübertragungshülse zur Ausbildung einer gasdichten Verbindung unter Vorspannung anliegt. Ferner kann die Dichtungskontur mit geringem Abstand zur Stirnseite des Absatzes an diesem ausgebildet sein, um das Einführen des Absatzes in die Wärmeübertragungshülse nicht zu behindern. Vorzugsweise dient als Dichtungskontur mindestens ein umlaufender radial nach außen abstehender Dichtungsbund mit rundem oder mehreckigem Querschnitt.

Bei einer besonders bevorzugten Ausführungsform ist an jeder der beiden Hülsen ein das der Wärmeübertragungshülse entgegengesetzte freie Ende bildender Abschnitt mittleren Durchmessers ausgebildet. Dieser Abschnitt mittleren Durchmessers dient bei der einen Hülse zum Befestigen eines hülsenartigen Mundstücks, während auf den Abschnitt der anderen Hülse ein Ansaugrohr aufgeschoben und an diesen befestigt ist. Durch Ausbilden der Abschnitte mittleren Durchmessers kann die erfindungsgemäße Inhalationseinrichtung auf einfache Weise an unterschiedliche Ausführungsformen angepasst werden, so beispielsweise an die Ausführungsform einer herkömmlichen Zigarette oder Zigarre. Das Mundstück oder auch das Ansaugrohr können gegebenenfalls auch mit Drosseln ausgestattet sein, um den Zugwiderstand der Inhalationseinrichtung, also den Strömungswiderstand, der das Ansaugen der Luft durch die Inhalationseinrichtung erschwert, an einen vorgegebenen Zugwiderstand anzupassen, beispielswese an den Zugwiderstand einer herkömmlichen Zigarette oder Zigarre. Als Drosselelemente können beispielsweise herkömmliche Filtereinsätze aus Acetat verwendet werden, wie sie bei herkömmlichen Zigaretten Verwendung finden. Bei der beschriebenen bevorzugten Ausführungsform sind vorzugsweise beide Hülsen identisch ausgebildet, wodurch die Teilevielfalt bei der Herstellung minimiert ist. Es ist jedoch auch denkbar, unterschiedliche Hülsen zu verwenden, beispielsweise eine Hülse, bei der der Abschnitt bereits in Form eines Mundstücks oder auch in Form eines Ansaugrohres ausgeführt ist.

Die beiden Hülsen sind vorzugsweise aus einem vorzugsweise elastischen Kunststoffmaterial gefertigt und durch eine Presspassung mit der Wärmeübertragungshülse fest und vorzugsweise gasdicht verbunden. Zum Befestigen können die Hülsen auch aus einem thermoplastischen Kunststoffmaterial gefertigt und durch Schweißen, vorzugsweise durch Ultraschallschweißen, fest mit der Wärmeübertragungshülse verbunden sein. Alternativ ist es auch möglich, die Hülsen durch Kleben mit der Wärmeübertragungshülse zu verbinden.

Der Strömungskanal hat über seine axiale Länge betrachtet eine gleichbleibende Querschnittsform. Bei einer besonders bevorzugten Ausführungsform variiert der Strömungskanal jedoch in seinen Innenabmessungen quer zu seiner axialen Länge betrachtet über seine axiale Länge gesehen. Dabei ist es von besonderem Vorteil, wenn der Strömungskanal im Bereich des Depots den Abschnitt mit der größte Innenabmessung aufweist, wodurch der von der angesaugten Luft durchströmte Querschnitt, und damit die Oberfläche aus der die Substanz aus dem Depot freigesetzt werden kann, maximal ist. Ferner ist es möglich, den Strömungskanal im Querschnitt zu verjüngen, um beispielsweise eine gewollte Verwirbelung des Luftstromes oder auch eine Erhöhung des Zugwiderstandes zu erreichen.

Im Auslieferungszustand ist die erfindungsgemäße Inhalationseinrichtung an ihren offenen Enden jeweils mit einer gasdicht schließenden Membran oder Folie verschlossen, die vor Benutzung der Inhalationseinrichtung zu entfernen bzw. zu durchstoßen oder aufzureißen sind. Die Membran oder Folie wird hierzu mit den die offenen Enden bildenden Abschnitten der Inhalationseinrichtung verklebt oder auch verschweißt.

Bei der Suche nach einem geeigneten Material für das Depot selbst wurde überraschend gefunden, dass sich Hybridstrukturen, also Strukturen aus unterschiedlichen Materialien, die eine hierarchische Porosität aufweisen, besonders gut eignen, um leichtflüchtige Substanzen, wie pharmazeutisch wirksamen Wirkstoffe, Aromastoffe und Geschmacksstoffe, einerseits gut zu speichern und andererseits bei entsprechender Wärmezufuhr in ausreichenden Mengen freizusetzen. Ferner wirkt die Hybridstruktur darüber hinaus als eine Art Filter.

Bei einer besonders bevorzugten Ausführungsform der erfindungsgemäßen Inhalationseinrichtung weist das Depot eine Hybridstruktur aus einem offenporigen makroporösen Stützgerüst auf, durch das der Luftstrom strömen kann, während im Stützgerüst vorgesehenen Poren zur Speicherung und Freisetzung leichtflüchtiger Substanzen, wie pharmazeutisch wirksamer Wirkstoffe, Aromastoffe und Geschmacksstoffe, dienen.

Vorteilhafterweise besteht das Stützgerüst aus dauerhaft miteinander verbundenen Partikeln oder Fasern, während die Poren in porösen Partikeln ausgebildet sind, welche im Stützgerüst immobilisiert gehalten sind. Bei dieser Lösung bestehen die porösen Partikel vorzugsweise aus einem anderen Material als das Stützgerüst.

Gemäß einer bevorzugten Ausführungsform werden die porösen Partikel zusammen mit dem partikulären Material (Mikropartikel, Fasern oder dergleichen) gemischt und dann das partikuläre Material miteinander zur Bildung eines offenporigen Stützgerüsts verbunden, wobei dadurch gleichzeitig die porösen Partikel im oder am Stützgerüst immobilisiert werden.

Besonders bevorzugt weisen die porösen Partikel im Vergleich zum Material, aus dem das Stützgerüst besteht, oder im Vergleich zu den Partikeln, aus denen das Stützgerüst gebildet wird, eine höhere Schmelztemperatur auf. Dadurch wird gewährleistet, dass bei einer thermischen Behandlung zur Verfestigung des Stützgerüsts die porösen Partikel nicht schmelzen oder deren Poren verkleben. Die porösen Partikel können somit in die Oberfläche der Stützgerüstpartikel beim Erweichen "eingebettet" werden.

Ein besonderer Vorteil der an der Oberfläche eines makroporösen Stützgerüsts dauerhaft durch Sintern befestigten porösen Partikeln mit hoher spezifischer Oberfläche und Affinität für Wirk- und Aromastoffe und deren Verwendung in einem Depot zur Substanz-Freisetzung in durchströmende Luft besteht darin, dass lediglich die jeweils zuvor gebundenen Substanzen freigesetzt werden. Die Fixierung der porösen Partikel am makroporösen Stützgerüst verhindert, dass die porösen Partikel vom Luftstrom mitgerissen werden können.

Alternativ oder auch ergänzend hierzu kann das Stützgerüst auch im wesentlichen aus einem anorganischen Material, wie Glas, Silikaten oder Alumosilikaten, gebildet sein, während die Poren im Material des Stützgerüstes selbst ausgebildet sind.

Die Menge und die Größe der porösen Partikel sind so gewählt, dass die Makroporen einerseits nicht verstopft werden und andererseits genügend Wirkstoff im Inneren der Partikel adsorbiert werden kann.

Das Stützgerüst ist vorzugsweise aus Partikeln, Fasern, partikulärem Material, oder Gemischen aus diesen Materialien aufgebaut. Das partikuläre Material oder Materialgemisch kann beispielsweise gesintert, angeschmolzen, geklebt oder anderweitig untereinander verbunden werden, so dass ein mechanisch stabiles offenporiges Stützgerüst entsteht.

Die Fixierung der porösen Partikeln an der Oberfläche des beispielsweise beim Sintern gebildeten makroporösen Stützgerüsts kann so erfolgen, dass die Poren des fixierten Partikels auf seiner von der Oberfläche des Stützgerüsts abgewandten Seite nicht verschlossen werden. Auch beim Befüllen des Depots wird das Verschließen dieser Poren vermieden.

Alternativ kann das Stützgerüst aber auch aus einer losen Schüttung aus partikulärem Material oder einem Materialgemisch bestehen, wobei das Stützgerüst in einem geeigneten Behälter des Depots vorliegt.

Das Depot kann auch durch ein Gemisch aus unterschiedlich großen Stützpartikeln oder Fasern bzw. aus Partikeln die aus unterschiedlichen Materialien bestehen, gebildet werden. Ebenso können verschiedene poröse Partikel gleichzeitig in dem Stützgerüst eingebettet werden, die sich in ihrer Größe und/oder Form und/oder Porengröße und/oder Material und/oder Affinität zu der zu speichernden Substanz unterscheiden. Die zur Herstellung des Depots verwendeten Partikel können ebenso ein Gemisch unterschiedlich beladener oder mit unterschiedlichen Substanzen beladener poröser Partikel sein.

Je nach Verwendungszweck wird die Länge und die Breite bzw. der Durchmesser des makroporösen Stützgerüstes in Abhängigkeit von der Flüchtigkeit des Wirkstoffes, der gewünschten Konzentration der freizusetzenden Substanz des das Depot verlassenden Luftstromes, der Beladungsmenge, dem Strömungswiderstand, sowie der dafür zu immobilisierenden Menge poröser Partikeln geeignet gewählt werden.

Ein für die erfindungsgemäße Inhalationseinrichtung geeignetes Depot ist beispielweise in der DE 10 2011 011 676.1 beschrieben, deren Inhalt hinsichtlich des Aufbaus des Depots hiermit einbezogen wird.

Nachfolgend wird die Erfindung anhand zweier Ausführungsbeispiele unter Bezugnahme auf die Zeichnung näher erläutert. Hierin zeigt:
- Fig. 1: eine Schnittansicht durch ein erstes Ausführungsbeispiel einer erfindungsgemäßen Inhalationseinrichtung,
- Fig. 2: eine schematische vergrößerte Darstellung eines Wirkstoff-Depots, das in der in Fig. 1 gezeigten erfindungsgemäßen Inhalationseinrichtung verwendet wird,
- Fig. 3: eine vergrößerte Darstellung eines Details der Inhalationseinrichtung aus Fig. 1, in der zwei an einer der Hülsen vorgesehene Dichtungsbunde vergrößert dargestellt sind, und
- Fig. 4: eine Schnittansicht durch ein zweites Ausführungsbeispiel einer erfindungsgemäßen Inhalationseinrichtung.

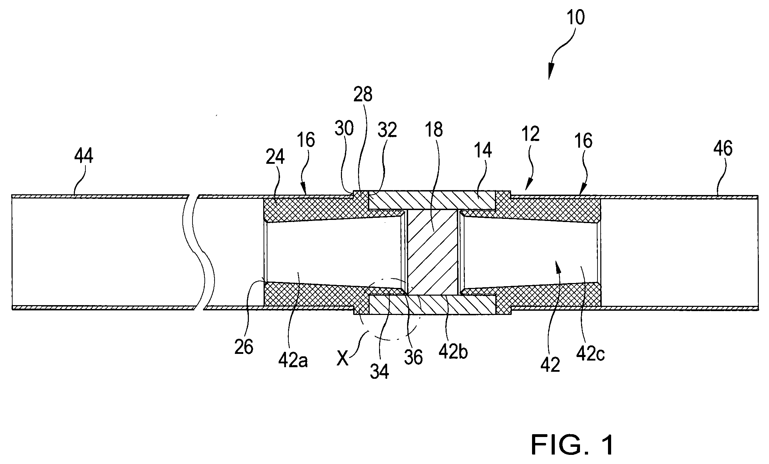

Fig. 1 zeigt eine Schnittansicht eines ersten Ausführungsbeispiels einer erfindungsgemäßen Inhalationseinrichtung 10. Die Inhalationseinrichtung 10 hat einen Grundkörper 12. Der Grundkörper 12 ist aus einer Wärmeübertragungshülse 14 und zwei identischen Hülsen 16 gebildet, welche fest mit der Wärmeübertragungshülse 14 verbunden sind.

Die Wärmeübertragungshülse 14 ist in Form eines Hohlzylinders ausgebildet und aus einem Werkstoff mit sehr hoher Wärmeleitfähigkeit gefertigt, beispielsweise aus Aluminium oder aus einer Aluminiumlegierung.

In der Wärmeübertragungshülse 14 ist ein Depot 18 aufgenommen, in dem mindestens eine bei Raumtemperatur flüchtige Substanz, beispielsweise eine freizusetzender Wirkstoff, ein Aromastoff oder ein Geschmacksstoff, gespeichert ist. Im vorliegenden Fall dient das Depot 18 zum Abspeichern einer vorgegebenen Menge einer pharmazeutisch wirksamen Substanz, beispielsweise Nikotin oder einem Nikotinderivat. Das Depot 18 ist in Form eines Zylinders ausgebildet und steht mit der Innenoberfläche der Wärmeübertragungshülse 14 in Berührung.

Aus dem durchschnittlichen Zugvolumen von 35 ml und der Zugzeit eines durchschnittlichen Benutzers von 1 bis 2 Sekunden ergibt sich eine sehr kurze Kontaktzeit der das Depot 18 durchströmenden Luft mit dem im Depot 18 gespeicherten Nikotin. Dabei korreliert der Übergang der freizusetzenden Substanz in die Gasphase in etwa mit der Kontaktfläche zwischen dem Depot 18 und der Luft.

Das gezeigte Depot 18 ist so in der Wärmeübertragungshülse 14 aufgenommen, dass Wärmeübertragungshülse 14 beim Halten der Inhalationseinrichtung 10 mit der Hand des Benutzers unmittelbar in Berührung ist. Die Handwärme wird dann an die Wärmeübertragungshülse 14 übertragen, welcher ihrerseits die Wärme nach innen an das Depot 18 weiterleitet. Durch das Erwärmen des Depots 18 wird die Freisetzungsrate der flüchtigen Substanz sowie gegebenenfalls im Depot 18 gespeicherter weiterer Substanzen, beispielsweise Aromastoffen, erhöht, so dass bei jedem Zug eine ausreichende Menge Substanz und Aromastoffe vom Benutzer konsumiert werden kann. Das Volumen der Wärmeübertragungshülse 14 wird über deren Wandstärke so angepasst, dass die Aufwärmzeit nach der Positionierung zwischen den Fingern möglichst schnell verläuft.

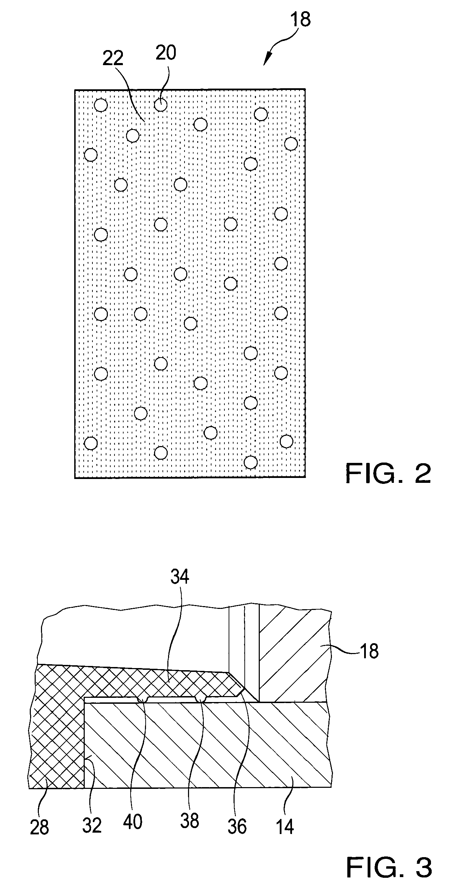

Fig. 2 zeigt den schematischen Aufbau des Depots 18 zur Speicherung der flüchtigen Substanzen. Das Depot 18 weist ein makroporöses Stützgerüst 20 auf, das aus gesinterten Partikeln gebildet ist. In dem Stützgerüst 20 sind Poren, Kavernen und Zwischenräume 22 ausgebildet, die nach dem Sintern erhalten geblieben sind. Die Kavernen und Zwischenräume 22 sind dabei so ausgebildet, dass das Stützgerüst 20 einen definierten Zugwiderstand für einen von einem Benutzer anzusaugenden Luftstrom aufweist.

Die Poren, Kavernen und Zwischenräume 22 bilden ein durchgängiges Netzwerk in dem poröse Partikel (nicht dargestellt) stabil und dauerhaft angehaftet bzw. eingebettet sind. Die porösen Partikel weisen Poren im Bereich von 1 nm bis 900 nm auf. An der Oberfläche der porösen Partikel, insbesondere auf deren inneren, von den Porenwänden gebildeten Oberflächen sind die flüchtigen Substanzen adsorbiert und stehen beim Ansaugen von Luft durch das Depot 18 im ständigen Austausch mit der durch das Stützgerüst 20 strömenden Luft.

Das hier beschriebene Depot 18 kann einerseits eine ausreichende Nikotinfreisetzung in die Atemluft gewährleisten und andererseits das Nikotin vor Oxidation schützen.

In jedes offene Ende der Wärmeübertragungshülse 14 ist jeweils eine der beiden aus Kunststoff gefertigten Hülse 16 eingesetzt. Die beiden Hülsen 16 sind identisch ausgebildet, so dass nachfolgend nur die in Fig. 1 links gezeigte Hülse 16 näher beschrieben wird.

Die rotationssymmetrische Hülse 16 hat an ihrem in Fig. 1 links gezeigten Ende einen Abschnitt 24 mittleren Außendurchmessers. Das in Fig. 1 links dargestellte Ende des Abschnittes 24 ist mit einer Fase 26 versehen.

An den Abschnitt 24, der sich etwa über zwei Drittel der axialen Länge der Hülse 16 erstreckt, schließt sich ein umlaufender Bund 28 größeren Durchmessers an, der sich radial nach außen erstreckt. Der Bund 28 hat in axialer Richtung gesehen eine in Fig. 1 links dargestellte erste Anlagefläch 30 und eine in Fig. 1 rechts dargestellte zweite Anlagefläche 32.

Die zweite Anlagefläche 32 des Bundes 28 geht in einen Absatz 34 kleineren Durchmessers über. Das freie Ende des Absatzes 34 ist gleichfalls mit einer Fase 36 versehen. Die Hülse 16 ist mit dem Absatz 34 in die hohlzylindrische Wärmeübertragungshülse 14 soweit eingeführt, bis die Wärmeübertragungshülse 14 mit ihrer Stirnseite an der zweiten Anlagefläche 32 des Bundes 28 anliegt. Der Außendurchmesser des Absatzes 34 ist dabei so bemessen, dass der Absatz 34 mit der Innenumfangsfläche der Wärmeübertragungshülse 14 eine Presspassung ausbildet, so dass die beiden Hülsen 16 fest und gasdicht mit der Wärmeübertragungshülse 14 verbunden sind.

In Fig. 3 ist in vergrößerter Darstellung der Übergang zwischen dem Absatz 34 und der Wärmeübertragungshülse 14 gezeigt. Wie Fig. 3 zu entnehmen ist, ist der Absatz 34 zusätzlich mit zwei umlaufenden Dichtungsbunden 38 und 40 versehen, welche unter Vorspannung an der Innenumfangsfläche der Wärmeübertragungshülse 14 anliegen.. Auf diese Weise wird eine gasdichte Verbindung zwischen der Hülse 16 und der Wärmeübertragungshülse 14 sichergestellt. In entsprechender Weise ist auch die in Fig. 1 rechts gezeigte, zur links dargestellten Hülse 16 identisch ausgebildete Hülse 16 mit der Wärmeübertragungshülse 14 gasdicht verbunden.

Die axiale Länge des Absatzes 34 jeder Hülse 16 ist so gewählt und auf die axiale Länge des Depots 18 abgestimmt, dass das Depot 18 bei zusammengesetzter Inhalationseinrichtung 10 mittig in der Wärmeübertragungshülse 14 angeordnet zwischen den Absätzen 34 der beiden Hülsen 16 eingespannt, zumindest aber zwischen diesen angeordnet ist.

Durch die Inhalationseinrichtung 10 erstreckt sich ferner ein Strömungskanal 42. Der Strömungskanal 42 ist in mehrere Abschnitte 42a bis 42c unterteilt, nämlich einen in der ersten Hülse 16 ausgebildeten sich hohlkegelförmig erweiternden ersten Abschnitt 42a, einen in der Wärmeübertragungshülse 14 ausgebildeten hohlzylindrischen zweiten Abschnitt 42b, in dem das Depot 18 aufgenommen ist, sowie einen in der zweiten Hülse 16 ausgebildeten sich hohlkegelförmig verjüngenden dritten Abschnitt 42c.

Auf das freie Ende des Abschnittes 24 mittleren Durchmessers der in Fig. 1 links gezeigten Hülse 16 ist ein aus Papier gefertigtes Ansaugrohr 44 aufgesteckt. Auf das freie Ende des Abschnittes 24 mittleren Durchmessers der in Fig. 1 rechts gezeigten Hülse 16 ist ein aus Papier gefertigtes Mundstück 46 aufgesteckt. Das Ansaugrohr 44 als auch das Mundstück 46, die die mit dem Strömungskanal 42 in Strömungsverbindung stehen, sind beide als Hohlzylinder ausgebildet und so bemessen, dass sie fest mit den Hülsen 16 verklemmt sind, ohne dass hierzu weitere Befestigungsmittel, wie Klebeverbindungen, vorgesehen sein müssen. Alternativ kann das Ansaugrohr 44 und/oder das Mundstück 46 auch an den Hülsen verklebt sein.

Die in Fig. 1 gezeigte Inhalationseinrichtung 10 ist aufgrund ihres Aufbaus verglichen mit den bekannten Lösung vergleichsweise einfach und ohne großen Aufwand montierbar. Ferner ist das Einsetzen des Depots 18 in die Wärmeübertragungshülse 14 vergleichsweise einfach.

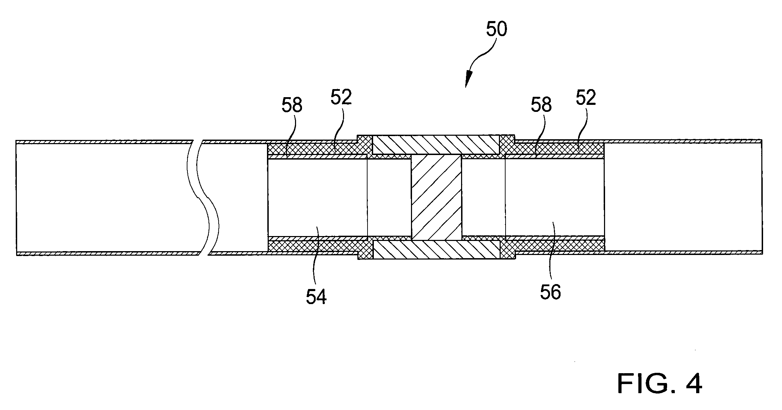

In Fig. 4 ist ein zweites Ausführungsbeispiel einer erfindungsgemäßen Inhalationseinrichtung 50 gezeigt. Der Aufbau der in Fig. 4 gezeigten Inhalationseinrichtung 50 entspricht im wesentlichem dem Aufbau der in Fig. 1 gezeigten Inhalationseinrichtung 10. Einziger Unterschied zwischen den beiden Ausführungsformen besteht in der Ausführung der beiden Hülsen 52. Die beiden identisch ausgebildeten Hülsen 52 haben anstelle eines sich hohlkegelförmig verjüngenden bzw. erweiternden Abschnitts 42a und 42c vgl. Fig. 1) jeweils einen hohlzylinderförmigen Abschnitt 54 als Teil des Strömungskanals 56, in den eine Metallhülse 58 eingesetzt ist. Mit Hilfe der Metallhülse 58 wird die gesamte Anordnung zusätzlich steifer, um die Widerstandsfähigkeit der Inhalationseinrichtung 50 insgesamt zu erhöhen.

### Bezugszeichenliste:

- 10: Inhalationseinrichtung
- 12: Grundkörper
- 14: Wärmeübertragungshülse
- 16: Hülsen
- 18: Depot
- 20: Stützstruktur
- 22: Poren
- 24: Abschnitt mittleren Durchmessers
- 26: Fase
- 28: Bund
- 30: erste Anlagefläche
- 32: zweite Anlagefläche
- 34: Absatz kleineren Durchmessers
- 36: Fase
- 38: Dichtungsbund
- 40: Dichtungsbund
- 42: Strömungskanal
- 42a: erster Abschnitt des Strömungskanals
- 42b: zweiter Abschnitt des Strömungskanals
- 42c: dritter Abschnitt des Strömungskanals
- 44: Ansaugrohr
- 46: Mundstück

- 50: Inhalationseinrichtung
- 52: Hülse
- 54: Abschnitt
- 56: Strömungskanal
- 58: Metallhülse

## Patentansprüche

1. Passive Inhalationseinrichtung, insbesondere Inhalationseinrichtung für pharmazeutische Inhaltsstoffe, rauchfreie kalte Zigarette, Zigarre oder Pfeife, zur Abgabe mindestens einer bei Raumtemperatur zumindest teilweise in die Gasphase übergehenden flüchtigen Substanz, bei der die für die Freisetzung der flüchtigen Substanz erforderliche Erwärmung im wesentlichen passiv durch die Körpertemperatur des Benutzers erfolgt, mit
einem sich durch einen Grundkörper (12) der Inhalationseinrichtung (10; 50) erstreckenden Strömungskanal (42), durch welchen der Benutzer bei Verwendung Luft ansaugt,
einem in dem Strömungskanal (42) angeordneten Depot (18), in dem die mindestens eine flüchtige Substanz bis zur Freisetzung gespeichert ist, und
einem mit dem Depot (18) zumindest abschnittsweise zur Wärmeübertragung in Berührung stehenden Wärmeübertragungselement (14), welches bei Benutzung die Körperwärme des Benutzers auf das Depot (18) zur Freisetzung der im Depot (18) gespeicherten Substanz überträgt, damit sich die aus dem Depot (18) freigesetzte flüchtige Substanz in der durch den Benutzer durch den Strömungskanal (42) angesaugten Luft verteilt,
**dadurch gekennzeichnet,**
**dass** der Grundkörper (12) zwei den Strömungskanal (42; 56) abschnittsweise bildende Hülsen (16; 52) aufweist, zwischen deren einander zugewandten Stirnseiten das Depot (18) angeordnet ist,
**dass** das als Teil des Grundkörpers (12) ausgebildete Wärmeübertragungselement eine einen weiteren Abschnitt des Strömungskanals (42; 56) begrenzende Wärmeübertragungshülse (14) ist, in der das Depot (18) gehalten ist, und
**dass** die Wärmeübertragungshülse (14) die beiden Hülsen (16; 52) starr miteinander verbindet und an diesen gasdicht befestigt ist.

2. Passive Inhalationseinrichtung gemäß Anspruch 1, **dadurch gekennzeichnet, dass** die Wärmeübertragungshülse (14) zumindest abschnittsweise aus einem Material mit hoher Wärmeleitfähigkeit gefertigt ist.

3. Passive Inhalationseinrichtung gemäß Anspruch 1 oder 2, **dadurch gekennzeichnet, dass** die beiden Hülsen (16; 52) jeweils einen Bund (28) größeren Durchmessers aufweisen, welcher in einen das freie Ende der jeweiligen Hülse (16; 52) bildenden Absatz (34) kleineren Durchmessers übergeht, wobei jede der Hülsen (16; 52) mit seinem Absatz (34) in die Wärmeübertragungshülse (14) eingeführt ist und sich mit seinem Bund (28) an der jeweiligen Stirnseite der Wärmeübertragungshülse (14) abstützt.

4. Passive Inhalationseinrichtung gemäß Anspruch 3, **dadurch gekennzeichnet, dass** an jedem Absatz (34) zumindest eine umlaufende Dichtungskontur (38, 40) ausgebildet ist, mit welcher der Absatz (34) an der Innenoberfläche der Wärmeübertragungshülse (14) zur Ausbildung einer gasdichten Verbindung unter Vorspannung anliegt.

5. Passive Inhalationseinrichtung gemäß Anspruch 4, **dadurch gekennzeichnet, dass** als Dichtungskontur mindestens ein umlaufender radial nach außen abstehender Dichtungsbund (38, 40) mit rundem oder mehreckigem Querschnitt dient.

6. Passive Inhalationseinrichtung gemäß einem der Ansprüche 1 bis 5, **dadurch gekennzeichnet, dass** an jeder der beiden Hülsen (16; 52) ein das andere freie Ende bildender zweiter Abschnitt (24) mittleren Durchmessers ausgebildet ist, und dass auf den zweiten Abschnitt (24) der einen Hülse (16; 52) ein hülsenartiges Mundstück (46) und auf den zweiten Abschnitt (24) der anderen Hülse (16; 52) ein Ansaugrohr (44) aufgeschoben und an diesen befestigt sind.

7. Passive Inhalationseinrichtung gemäß einem der Ansprüche 1 bis 6, **dadurch gekennzeichnet, dass** die beiden Hülsen (16; 52) aus einem elastischen Kunststoffmaterial gefertigt und durch eine Presspassung mit der Wärmeübertragungshülse (14) fest und vorzugsweise gasdicht verbunden sind.

8. Passive Inhalationseinrichtung gemäß einem der Ansprüche 1 bis 7, **dadurch gekennzeichnet, dass** der Strömungskanal (42) in seinen Innenabmessungen quer zu seiner axialen Länge betrachtet über seine axiale Länge gesehen variiert und vorzugsweise im Bereich des Depots (18) den Abschnitt mit der größte Innenabmessung aufweist.

9. Passive Inhalationseinrichtung gemäß einem der vorhergehenden Ansprüche, **dadurch gekennzeichnet, dass** die offenen Enden der Inhalationseinrichtung (10; 50) jeweils mit einer die offenen Enden gasdicht schließenden Membran oder Folie verschlossen sind, die vor Benutzung der Inhalationseinrichtung (10; 50) zu entfernen bzw. zu zerstören sind.

10. Passive Inhalationseinrichtung gemäß einem der vorhergehenden Ansprüche, **dadurch gekennzeichnet, dass** das Depot (18) eine Hybridstruktur aus einem offenporigen makroporösen Stützgerüst (20) ist, durch das der Luftstrom strömen kann, und im Stützgerüst (20) vorgesehenen Poren zur Speicherung und Freisetzung der Substanz aufweist.

11. Passive Inhalationseinrichtung gemäß Anspruch 10, **dadurch gekennzeichnet, dass** das Stützgerüst (20) aus dauerhaft miteinander verbundenen Partikeln oder Fasern besteht und die Poren in porösen Partikeln ausgebildet sind, welche im Stützgerüst immobilisiert gehalten sind.

12. Passive Inhalationseinrichtung gemäß Anspruch 11, **dadurch gekennzeichnet, dass** die porösen Partikel aus einem anderen Material als das Stützgerüst (20) bestehen.

13. Passive Inhalationseinrichtung gemäß Anspruch 11 oder 12, **dadurch gekennzeichnet, dass** das Stützgerüst (20) im wesentlichen aus einem anorganischen Material, wie Glas, Silikaten oder Alumosilikaten, besteht und die Poren im Material des Stützgerüstes (20) ausgebildet sind.

14. Passive Inhalationseinrichtung gemäß einem der vorhergehenden Ansprüche, **dadurch gekennzeichnet, dass** als flüchtige Substanz ein pharmazeutisch wirksamer Wirkstoff, ein Aromastoff oder eine Mischung aus mindestens einem Wirkstoff und mindestens einem Aromastoff im Depot (18) gespeichert ist bzw. sind.

## Claims

1. A passive inhalation device, particularly an inhalation device for pharmaceutical substances, smokeless cold cigarette, cigar, or pipe, for delivering at least one volatile substance transitioning at least partially into the gaseous phase at room temperature, the heating required for releasing the volatile substance occurring substantially passively by the body temperature of the user, having
a flow channel (42) extending through a base body (12) of the inhalation device (10; 50), through which the user draws air during use,
a depot (18) disposed in the flow channel (42), in which the at least one volatile substance is stored until released, and a heat transfer element (14) contacting the depot (18) at least in segments for transferring heat, said element transferring the body heat of the user to the depot (18) during use for releasing the substance stored in the depot (18), so that the volatile substance released from the depot (18) disperses into the air drawn in by the user through the flow channel (42), **characterized in that**
the base body (12) comprises two sleeves (16; 52) forming the flow channel (42; 56) in segments, wherein the depot (18) is disposed between the end faces thereof facing toward each other,
that the heat transfer element implemented as part of the base body (12) is a heat transfer sleeve (14) bounding a further segment of the flow channel (42; 56), in which the depot (18) is held, and
that the heat transfer sleeve (14) connects the two sleeves (16; 52) rigidly to each other and is connected thereto in a gas-tight manner.

2. The passive inhalation device according to claim 1, **characterized in that** the heat transfer sleeve (14) is made at least in segments of a material having high thermal conductivity.

3. The passive inhalation device according to claim 1 or 2, **characterized in that** the two sleeves (16; 52) each comprise a flange (28) of greater diameter transitioning into an offset (34) of lesser diameter forming the free end of each sleeve (16; 52), wherein the offset (34) of each of the sleeves (16; 52) is inserted into the heat transfer sleeve (14) and the flange (28) thereof is supported against the corresponding end face of the heat transfer sleeve (14).

4. The passive inhalation device according to claim 3, **characterized in that** at least one circumferential sealing contour (38, 40) is implemented on each offset (34), by means of which the offset (34) contacts the inner surface of the heat transfer sleeve (14) for forming a pretensioned gas-tight connection.

5. The passive inhalation device according to claim 4, **characterized in that** at least one circumferential sealing flange (38, 40) having a round or polygonal cross section and protruding radially outward serves as a sealing contour.

6. The passive inhalation device according to any one of the claims 1 through 5, **characterized in that** a second segment (24) of medium diameter is implemented on each of the two sleeves (16; 52) and forms the other free end thereof, and that a sleeve-like mouthpiece (46) is inserted onto the second segment (24) of one of the sleeves (16; 52) and an intake tube (44) is inserted onto the second segment (24) of the other sleeve (16; 52) and are each attached to the same.

7. The passive inhalation device according to any one of the claims 1 through 6, **characterized in that** the two sleeves (16; 52) are made of an elastic plastic material and are firmly connected to the heat transfer sleeve (14) by means of a press fit, preferably in a gas-tight manner.

8. The passive inhalation device according to any one of the claims 1 through 7, **characterized in that** the inner dimensions of the flow channel (42) transverse to the axial length thereof vary along the axial length thereof and preferably comprises the segment having the largest inner dimensions in the region of the depot (18).

9. The passive inhalation device according to any one of the preceding claims, **characterized in that** the open ends of the inhalation device (10; 50) are each closed off by means of a membrane or foil closing off the open ends in a gas-tight manner and must be removed or destroyed prior to using the inhalation device (10; 50).

10. The passive inhalation device according to any one of the preceding claims, **characterized in that** the depot (18) is a hybrid structure of an open-pored macroporous support matrix (20) through which the airflow can flow and pores provided in the support matrix (20) for storing and releasing the substance.

11. The passive inhalation device according to claim 10, **characterized in that** the support matrix (20) is made of particles or fibers permanently connected to each other, and the pores are implemented in porous particles held immobilized in the support matrix.

12. The passive inhalation device according to claim 11, **characterized in that** the porous particles are made of a different material than the support matrix (20).

13. The passive inhalation device according to claim 11 or 12, **characterized in that** the support matrix (20) is substantially made of an inorganic material such as glass, silicates, or alumosilicates, and the pores are implemented in the material of the support matrix (20).

14. The passive inhalation device according to any one of the preceding claims, **characterized in that** a pharmaceutically active substance, a flavoring agent, or a mixture of at least one active substance and at least one flavoring agent is or are stored in the depot (18) as the volatile substance.

## Revendications

1. Dispositif d'inhalation passif, en particulier dispositif d'inhalation passif pour ingrédients pharmaceutiques, cigarette froide sans fumée, cigare ou pipe, pour la distribution d'au moins une substance volatile passant en phase gazeuse au moins partiellement à la température ambiante, dans lequel l'échauffement nécessaire pour le dégagement de la substance volatile s'effectue essentiellement de manière passive sous l'effet de la température corporelle de l'utilisateur, avec
un canal d'écoulement (42) s'étendant à travers un corps de base (12) du dispositif d'inhalation (10 ; 50) à travers lequel l'utilisateur aspire l'air lors de l'utilisation,
un dépôt (18) disposé dans le canal d'écoulement (42), dans lequel l'au moins une substance volatile est accumulée jusqu'au dégagement,
et un élément de transfert de chaleur (14) en contact avec le dépôt (18) au moins en certaines sections pour le transfert de chaleur, lequel, lors de l'utilisation, transmet la chaleur corporelle de l'utilisateur sur le dépôt (18) en vue du dégagement de la substance accumulée dans le dépôt (18), afin que la substance volatile dégagée du dépôt (18) se répande dans l'air aspiré par l'utilisateur à travers le canal d'écoulement (42),
**caractérisé en ce que**
le corps de base (12) comporte deux douilles (16 ; 52) formant le canal d'écoulement (42 ; 56) en certaines sections, le dépôt (18) étant disposé entre leurs faces frontales orientées l'une vers l'autre,
l'élément de transfert de chaleur se présentant sous la forme d'une partie du corps de base (12) est une douille de transfert de chaleur (14) limitant une section supplémentaire du canal d'écoulement (42 ; 56), dans laquelle est fixé le dépôt (18), et la douille de transfert de chaleur (14) relie rigidement les deux douilles (16 ; 52) et qu'elle est fixée à ces dernières de manière étanche aux gaz.

2. Dispositif d'inhalation passif selon la revendication 1, **caractérisé en ce que** la douille de transfert de chaleur (14) est fabriquée au moins en certaines sections en un matériau à haute conductivité thermique.

3. Dispositif d'inhalation passif selon la revendication 1 ou 2, **caractérisé en ce que** les deux douilles (16 ; 52) présentent respectivement une collerette (28) de diamètre plus élevé, laquelle se prolonge dans un épaulement (34) de diamètre plus faible formant l'extrémité libre de chacune des douilles (16 ; 52), sachant que chacune des douilles (16 ; 52) est introduite par son épaulement (34) dans la douille de transfert de chaleur (14) et s'appuie par sa collerette (28) sur la face frontale respective de la douille de transfert de chaleur (14).

4. Dispositif d'inhalation passif selon la revendication 3, **caractérisé en ce qu'**au moins un contour d'étanchéité (38, 40) circonférentiel est formé à chaque épaulement (34), par laquelle l'épaulement (34) adhère à la surface supérieure intérieure de la douille de transfert de chaleur (14) pour former sous précontrainte une liaison étanche aux gaz.

5. Dispositif d'inhalation passif selon la revendication 4, **caractérisé en ce qu'**au moins un bourrelet d'étanchéité (38, 40) circonférentiel en saillie radiale vers l'extérieur et à section transversale ronde ou polygonale sert de contour d'étanchéité.

6. Dispositif d'inhalation passif selon l'une des revendications 1 à 5, **caractérisé en ce qu'**une seconde section (24) de diamètre moyen, formant l'autre extrémité libre, est formée à chacune des deux douilles (16 ; 52), et qu'un embout buccal (46) de type douille est glissé et fixé sur la seconde section (24) de l'une des douilles (16 ; 52) et un tube d'aspiration (44) est glissé et fixé sur la seconde section (24) de l'autre douille (16 ; 52).

7. Dispositif d'inhalation passif selon l'une des revendications 1 bis 6, **caractérisé en ce que** les deux douilles (16 ; 52) sont fabriqués en une matière plastique élastique et assemblées rigidement, de préférence de manière étanche aux gaz, avec la douille de transfert de chaleur (14) par ajustement à serrage.

8. Dispositif d'inhalation passif selon l'une des revendications 1 à 7, **caractérisé en ce que** le canal d'écoulement (42), par ses dimensions intérieures vues perpendiculairement à sa longueur axiale, varie par sa longueur axiale et présente de préférence dans la zone du dépôt (18) la section avec les plus grandes dimensions intérieures.

9. Dispositif d'inhalation passif selon l'une des revendications précédentes, **caractérisé en ce que** les extrémités ouvertes du dispositif d'inhalation (10 ; 50) sont obturées respectivement par une membrane ou une pellicule bouchant de manière étanche aux gaz lesdites extrémités ouvertes, laquelle doit être supprimée ou détruite avant l'utilisation du dispositif d'inhalation (10 ; 50).

10. Dispositif d'inhalation passif selon l'une des revendications précédentes, **caractérisé en ce que** le dépôt (18) est une structure hybride composée d'une armature (20) macroporeuse à pores ouverts, à travers laquelle peut circuler l'écoulement d'air, et de pores prévus dans l'armature (20) pour l'accumulation et le dégagement de la substance.

11. Dispositif d'inhalation passif selon la revendication 10, **caractérisé en ce que** l'armature (20) se compose de particules ou de fibres reliées durablement les unes avec les autres et les pores sont formés dans des particules poreuses, qui sont fixées de manière immobile dans l'armature.

12. Dispositif d'inhalation passif selon la revendication 11, **caractérisé en ce que** les particules poreuses se composent d'une matière différente de celle de l'armature (20).

13. Dispositif d'inhalation passif selon la revendication 11 ou 12, **caractérisé en ce que** l'armature (20) se compose essentiellement d'une matière inorganique, telle que le verre, les silicates ou les aluminosilicates, et les pores sont formés dans la matière de l'armature (20).

14. Dispositif d'inhalation passif selon l'une des revendications précédentes, **caractérisé en ce qu'**une substance pharmaceutiquement active, une substance aromatique ou un mélange d'au moins une substance active et d'au moins une substance aromatique est/sont accumulée(s) dans le dépôt (18).
